# EUROPÄISCHE PATENTSCHRIFT

(11) **EP 1 098 669 B1**
(45) Veröffentlichungstag und Bekanntmachung des Hinweises auf die Patenterteilung: **29.01.2003**
(21) Anmeldenummer: 99920725.1
(22) Anmeldetag: 21.04.1999
(51) Int. Cl.: A61L 2/26

(54) **TELLERFÖRMIGE FILTERHALTERUNG FÜR EINEN STERILISIERBEHÄLTER**
PLATE-SHAPED FILTER HOLDER FOR A STERILIZATION VESSEL
FIXATION DE FILTRE BOMBEE POUR RECIPIENT DE STERILISATION

(30) Priorität: 21.07.1998 DE 19832823
(43) Veröffentlichungstag der Anmeldung: 16.05.2001
(73) Patentinhaber: Aesculap AG & Co. KG, 78532 Tuttlingen (DE)
(72) Erfinder: GABELE, Lorenz, D-88605 Sauldorf (DE); SCHWANKE, Wolfgang, D-78604 Rietheim-Weilheim (DE)
(74) Vertreter: Böhme, Ulrich, Dr. Dipl.-Phys.
(86) Internationale Anmeldenummer: EP9902682
(87) Internationale Veröffentlichungsnummer: WO00004933

(56) Entgegenhaltungen:
- DE-A- 3 710 049
- US-A- 5 736 043

## Beschreibung

Die Erfindung betrifft eine tellerförmige Filterhalterung für einen Sterilisierbehälter mit einem umlaufenden äußeren Andruckrand, einem zentralen Andruckbereich, einer Befestigungseinrichtung zur Festlegung der Filterhalterung am Sterilisierbehälter und mit zwischen sich Durchbrechungen frei lassenden Stegen zwischen dem äußeren Andruckrand und dem zentralen Andruckbereich.

Eine solche Filterhalterung für einen Sterilisierbehälter ist beispielsweise aus der DE 37 10 049 B1 bekannt. Mit Hilfe der tellerförmigen Filterhalterung können flächige Filtermaterialien, beispielsweise Papierfilter oder Textilfilter, dicht an Durchbrechungen im Sterilisierbehälter angedrückt werden, beispielsweise befinden sich derartige Durchbrechungen im Deckel eines Sterilisierbehälters, in einer Seitenwand oder im Bodenbereich desselben. Zur dauerhaften Festlegung der Filterlage ist es notwendig, die Filterhalterung federnd anzudrükken, und dies erfolgt bei bekannten Anordnungen durch eine zentrale Befestigungseinrichtung, die die Filterhalterung unter elastischer Verformung gegen die Filterlage drückt. Eine Abdichtung erfolgt bei der bekannten Filterhalterung einmal durch eine Dichtung längs des äußeren Andruckrandes und zum anderen durch eine mittlere Dichtung im zentralen Andruckbereich. Die bekannten Filterhalterungen verbinden den äußeren Andruckrand mit dem zentralen Andruckbereich über radiale Stege, die zwischen sich Durchbrechungen freilassen, durch die der Gasaustausch erfolgen kann.

Es hat sich herausgestellt, daß in derartigen Filterhalterungen Spannungen auftreten können, die zu einer unerwünschten Verformung der Filterhalterung führen. Derartige Verspannungen entstehen beispielsweise beim Einstanzen der Durchbrechungen oder bei Tiefziehvorgängen, bei denen die Filterhalterung in die gewünschte Form gebracht wird. Diese unerwünschten Verformungen führen zu einer ungleichmäßigen Anlage der Filterhalterung an der Filterlage und damit zu einer gegebenenfalls nicht immer gesicherten Abdichtung. Um dies zu vermeiden, ist es notwendig, die Verspannungen durch geeignete Nachbearbeitung der Filterhalterungen zu beseitigen, beispielsweise durch eine Wärmebehandlung.

Es ist Aufgabe der Erfindung, eine gattungsgemäße Filterhalterung so auszubilden, daß unerwünschte Verspannungen mit Sicherheit vermieden werden.

Diese Aufgabe wird bei einer Filterhalterung der eingangs beschriebenen Art erfindungsgemäß dadurch gelöst, daß die Stege zumindest abschnittsweise in einer Richtung verlaufen, die eine Komponente in Umfangsrichtung aufweist. Es hat sich überraschenderweise herausgestellt, daß keine Verspannungen auftreten, wenn dafür Sorge getragen wird, daß die Stege zwischen äußerem Andruckrand und zentralem Andruckbereich nicht exakt in radialer Richtung verlaufen, sondern wenn diese Stege zumindest abschnittsweise in Umfangsrichtung verlaufen oder zumindest in einer Richtung, die eine Komponente in Umfangsrichtung aufweist. Durch einen solchen Verlauf der Stege kann das Material in Umfangsrichtung nachfließen und bei der Herstellung eventuell auftretende Spannungen bereits beim Auftreten derartiger Spannungen wieder abbauen. Dies ist bei Stegen, die ausschließlich in radialer Richtung verlaufen, nicht möglich.

Die Stege können dabei geometrisch sehr unterschiedlich ausgebildet sein, immer vorausgesetzt, daß sie zumindest abschnittsweise eine in Umfangsrichtung weisende Komponente aufweisen.

Beispielsweise kann vorgesehen sein, daß die Stege zwischen zentralem Bereich und äußerem Andruckrand bogenförmig verlaufen.

Es ist auch möglich, daß die Stege zwischen zentralem Bereich und äußerem Andruckrand geradlinig verlaufen, wobei sie dann gegenüber der Radialrichtung einen Winkel einschließen.

Die Stege können über ihre gesamte Länge mit einer in Umfangsrichtung gleichgerichteten Komponente verlaufen, es ist aber bei einer abgewandelten Ausführungsform auch möglich, daß die in Umfangsrichtung verlaufende Komponente des Steges über dessen Länge ihre Richtung ändert.

Beispielsweise können die Stege entlang ihrer Längsausdehnung wellen- oder zickzackförmig verlaufen.

Bei einer besonders bevorzugten Ausführungsform ist vorgesehen, daß die Stege entlang ihrer Längsausdehnung Abschnitte aufweisen, die abwechselnd radial und in Umfangsrichtung verlaufen.

Die Stege können zwischen dem äußeren Andruckrand und dem zentralen Andruckbereich als selbständige Bauteile ausgebildet sein, es ist aber auch möglich, daß benachbarte Stege durch Verbindungsstege miteinander verbunden sind. Dadurch wird die Festigkeit der Filterhalterung erhöht, trotzdem werden unerwünschte Verspannungen wegen der Umfangskomponenten im Stegverlauf vermieden.

Bei einer besonders bevorzugten Ausführungsform ist vorgesehen, daß zwischen dem zentralen Andruckbereich und dem äußeren Andruckrand mindestens ein in Umfangsrichtung umlaufender Mittelsteg angeordnet ist und daß zwischen diesem und dem äußeren Andruckrand, dem inneren Andruckbereich oder einem benachbarten Mittelsteg Verbindungsstege mit radialer Komponente angeordnet sind, die auf gegenüberliegenden Seiten eines Mittelsteges in Umfangsrichtung gegeneinander versetzt in diesen einmünden. Insbesondere können die Verbindungsstege in radialer Richtung verlaufen. Man erhält dann eine Ausbildung, bei der zwar radial verlaufende Stege zwischen dem zentralen Andruckbereich und dem äußeren Andruckrand verlaufen, diese erstrecken sich aber nicht über den gesamten Abstand zwischen dem äußeren Andruckrand und dem zentralen Andruckbereich, sondern nur über einen Teilbereich, nämlich bis zum nächsten Mittelsteg, und dieser Mittelsteg verläuft in Umfangsrichtung, so daß insgesamt die Stegverbindung zwischen äußerem Andruckrand und zentralem Andruckbereich abwechselnd radial und in Umfangsrichtung verläuft. Auch dadurch ist sichergestellt, daß unerwünschte Verspannungen vermieden werden.

Zur sicheren Festlegung des Filtermaterials ist es günstig, wenn am äußeren Andruckrand eine Ringdichtung gehalten ist, außerdem kann auch am zentralen Andruckbereich eine elastisch verformbare Andruckdichtung angeordnet sein.

Bei einer besonders bevorzugten Ausführungsform ist diese Andruckdichtung eine Lippendichtung mit einer an den zentralen Andruckbereich andrückbaren Dichtlippe. Eine solche Dichtlippe läßt sich in relativ großem Umfang verformen, so daß auch bei Dichtmaterial unterschiedlicher Dicke in jedem Fall eine sichere Anlage im zentralen Bereich gewährleistet ist, auch wenn die Filterhalterung in allen Fällen denselben Abstand von dem Sterilisierbehälter einnimmt. Abstandstoleranzen werden durch die in großem Maße verformbare Dichtlippe aufgenommen.

Es ist günstig, wenn die Lippendichtung einen ringförmigen Lagerkörper aufweist, an den die ringförmige Dichtlippe angeformt ist.

Insbesondere kann vorgesehen sein, daß der Lagerkörper eine Ausnehmung aufweist, in welche die Lippendichtung eintaucht, wenn sie vollständig gegen den zentralen Andruckbereich gedrückt wird.

Eine besonders einfache Festlegung dieser Filterhalterung am Sterilisierbehälter ergibt sich, wenn gemäß einer bevorzugten Ausführungsform vorgesehen wird, daß die Befestigungseinrichtung ein Gehäuse mit zwei in Umfangsrichtung federnd gegeneinander schiebbaren Riegelkörpern aufweist, die in einer unverschobenen Verriegelungsstellung ein die Filterhalterung durchgreifendes Halteglied des Sterilisierbehälters hintergreifen und dieses in einer verschobenen Lösestellung freigeben, daß das Gehäuse am Filterhalter im zentralen Andruckbereich durch eine Drehbewegung festlegbar ist, bei der Vorsprünge und Rücksprünge an der Filterhalterung bzw. am Gehäuse sich hintergreifen, und daß mindestens eine lösbare Raste vorgesehen ist, die bei am Filterhalter festgelegtem Gehäuse dieses gegen eine Verdrehung relativ zum Filterhalter sichert. Eine solche Befestigungseinrichtung mit zwei gegeneinander verschiebbaren Riegelkörpern ist bei gattungsgemäßen Filterhaltern an sich bekannt (DE 37 10 049 B1), jedoch wird in diesem Falle das Gehäuse dauerhaft mit dem Filterhalter verbunden, beispielsweise durch Vernieten, Verschrauben, etc. Bei der beschriebenen Konstruktion jedoch wird das Gehäuse durch eine Drehbewegung nach Art eines Bajonettverschlusses mit dem Filterhalter verbunden und durch die lösbare Raste gegen ein unbeabsichtigtes Lösen gesichert. Es ist daher möglich, ohne Verwendung eines Werkzeuges und ohne Verwendung zusätzlicher Teile das Gehäuse mit den beiden Riegelkörpern an der Filterhalterung zu befestigen.

Besonders vorteilhaft ist es dabei, wenn die Raste durch einen der beiden Riegelkörper gebildet wird, der das Gehäuse in seiner Verriegelungsstellung gegen eine Verdrehung sichert und in seiner Lösestellung freigibt. Es ist bei dieser Ausgestaltung nicht notwendig, eine separate Raste vorzusehen, sondern der gegen Federkraft verschiebbare Riegelkörper im Gehäuse übernimmt zusätzlich die Aufgabe einer Raste, die das Gehäuse gegen unbeabsichtigtes Verdrehen und Lösen sichert.

Besonders vorteilhaft ist es weiterhin, wenn der andere Riegelkörper das Gehäuse in seiner Lösestellung gegen eine Verdrehung sichert und in seiner Verriegelungsstellung freigibt. Dadurch ist sichergestellt, daß beim Verschieben der Riegelkörper in die Lösestellung, bei der also die Filterhalterung das Halteglied des Sterilisierbehälters freigibt und vom Sterilisierbehälter abgenommen werden kann, das Gehäuse nicht unbeabsichtigt verdreht und von der Filterhalterung abgenommen werden kann. Zum Verdrehen des Gehäuses muß einer der beiden Riegelkörper in die Lösestellung verschoben werden, der andere Riegelkörper muß aber in der Verriegelungsstellung verbleiben. Somit ist eine Verdrehung des Gehäuses weder dann möglich, wenn beide Riegelkörper sich in der Verriegelungsstellung befinden, noch dann, wenn sich beide Riegelkörper in der Lösestellung befinden. Nur diese beiden Stellungen werden bei der normalen Betätigung der Filterhalterung auftreten, da die beiden Riegelkörper durch diametralen Druck von der Verriegelungsstellung in die Lösestellung verschoben werden. Wenn dagegen das Gehäuse gegenüber der Filterhalterung verdreht werden soll, um das Gehäuse abzunehmen, muß gezielt dafür Sorge getragen werden, daß nur einer der beiden Riegelkörper verschoben wird.

Dabei ist bei einer besonders bevorzugten Ausführungsform vorgesehen, daß Nockenbahnen und in diesen geführte Vorsprünge an dem zentralen Andruckbereich bzw. den Riegelkörpern zusammenwirken und dadurch ein Verdrehen des Gehäuses relativ zur Filterhalterung nur in den bestimmten Stellungen der Riegelkörper erlauben.

So kann beispielsweise vorgesehen sein, daß eine Nokkenbahn für den das Gehäuse in seiner Verriegelungsstellung sichernden Riegelkörper einen radial nach innen gerichteten Abschnitt und einen sich an dessen inneres Ende anschließenden, neben diesem Abschnitt nach außen verlaufenden Abschnitt aufweist. Mit einem solchen Abschnitt ist sichergestellt, daß eine Verdrehung des Gehäuses relativ zur Filterhalterung nur möglich ist, wenn der entsprechende Riegelkörper in die Lösestellung eingeschoben ist.

Weiterhin ist es günstig, wenn eine Nockenbahn für den das Gehäuse in seiner Freigabestellung sichernden Riegelkörper einen radial nach innen gerichteten Abschnitt und einen sich an dessen äußeres Ende anschließenden, in Umfangsrichtung verlaufenden Abschnitt aufweist. Dieser Riegelkörper ermöglicht somit ein Verdrehen des Gehäuses nur dann, wenn er selbst in der Verriegelungsstellung steht, bei allen anderen Stellungen dagegen wird eine Drehung des Gehäuses relativ zur Filterhalterung behindert.

Insbesondere kann vorgesehen sein, daß die radial nach innen gerichteten Abschnitte der Nockenbahnen und die in die Nockenbahnen eingreifenden Vorsprünge bezüglich der Drehachse des Gehäuses spiegelsymmetrisch angeordnet sind. Dadurch wird es möglich, die Nockenbahnen und die Vorsprünge so anzuordnen, daß das Gehäuse in zwei um 180° verdrehten Positionen auf die Filterhalterung aufgesetzt werden kann, beide Riegelkörper können je nach Orientierung der Aufsetzung des Gehäuses dann die Rolle des Riegelkörpers übernehmen, der das Gehäuse in der Lösestellung bzw. in der Verriegelungsstellung gegen eine Drehung sichert, d.h. man kann einen vollständig symmetrischen Aufbau der Riegelkörper benutzen und muß beim Aufsetzen des Gehäuses nicht darauf achten, welcher Vorsprung in welche Nockenbahn eintaucht.

Dazu ist es auch günstig, wenn die Endpunkte der sich an die radial nach innen gerichteten Abschnitte anschließenden Abschnitte bezüglich der Drehachse des Gehäuses spiegelsymmetrisch angeordnet sind.

Die nachfolgende Beschreibung bevorzugter Ausführungsformen der Erfindung dient im Zusammenhang mit der Zeichnung der näheren Erläuterung. Es zeigen:
- Figur 1:: eine perspektivische Ansicht der Innenseite eines Sterilisierbehälterdeckels mit einer Filterhalterung mit abgenommenem Gehäuse der Befestigungseinrichtung;
- Figur 2:: eine Draufsicht auf die Filterhalterung der Figur 1 mit aufgesetztem Gehäuse der Befestigungseinrichtung;
- Figur 3:: eine Schnittansicht längs Linie 3-3 in Figur 2;
- Figur 4:: eine vergrößerte Detailansicht der in die Filterhalterung eingesetzten zentralen Dichtung im unverformten Zustand,
- Figur 5:: eine Teilansicht eines weiteren bevorzugten Ausführungsbeispiels einer Filterhaiterung mit geradlinigen Stegen;
- Figur 6:: eine Ansicht ähnlich Figur 5 mit geradlinigen Stegen und einem Mittelsteg;
- Figur 7:: eine Ansicht ähnlich Figur 5 mit gebogenen Stegen;
- Figur 8:: eine Ansicht ähnlich Figur 5 mit gebogenen, tangential in den äußeren Anlagerand einmündenden Stegen;
- Figur 9:: eine Ansicht ähnlich Figur 5 mit gewellten Stegen;
- Figur 10:: eine Ansicht ähnlich Figur 5 mit zickzackförmigen Stegen;
- Figur 11:: eine Ansicht ähnlich Figur 5 mit radialen Stegen und einem Mittelsteg;
- Figur 12:: eine Ansicht ähnlich Figur 5 mit radialen Stegen und zwei Mittelstegen;
- Figur 13:: eine Querschnittansicht längs Linie 13-13 in Figur 14 des Gehäuses der Befestigungseinrichtung mit zwei darin gelagerten Riegelkörpern;
- Figur 14:: eine Schnittansicht längs Linie 14-14 in Figur 13;
- Figur 15:: eine Schnittansicht längs Linie 15-15 in Figur 13;
- Figur 16:: eine Schnittansicht ähnlich Figur 13 mit in Lösestellung verschobenen Riegelkörpern und
- Figur 17:: eine Ansicht ähnlich Figur 13 mit einem gegenüber der Filterhalterung verdrehten und von diesem abnehmbaren Gehäuse.

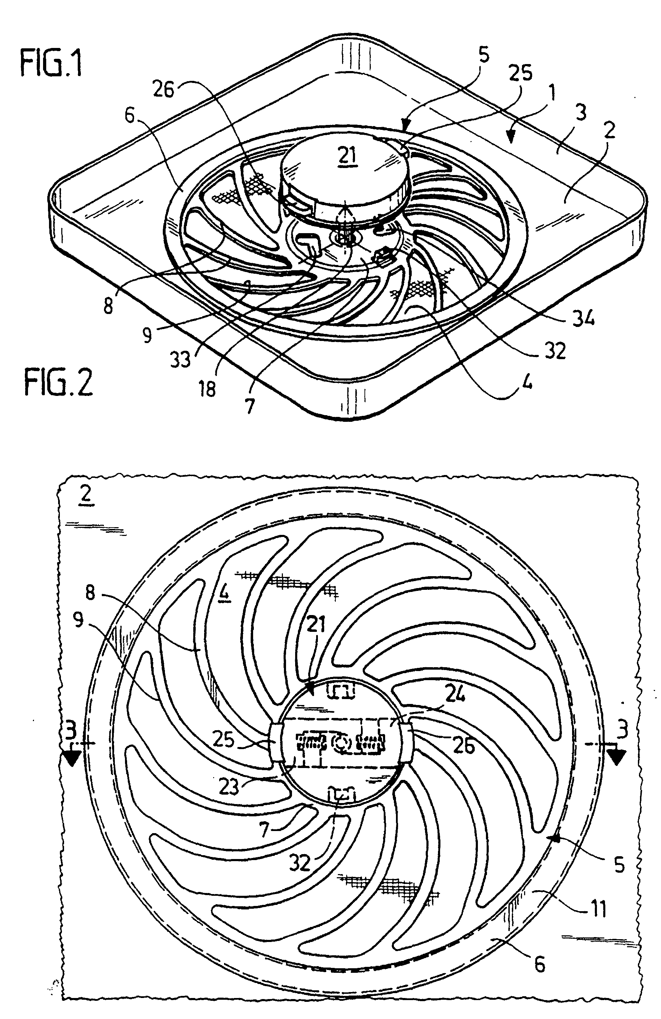

In Figur 1 ist ein umgekehrter Deckel 1 eines Sterilisierbehälters dargestellt, der eine ebene Oberseite 2 und einen sich nach unten daran anschließenden Rand 3 aufweist. Die Oberseite 2 weist Durchbrechungen auf, die durch eine flächige Filterlage 4 abgedeckt sind, beispielsweise durch ein Filterpapier oder durch ein textiles Flächenfilter.

Diese Filterlage 4 wird mittels einer Filterhalterung 5 gegen die Innenseite des Deckels 1 angedrückt, dazu weist diese Filterhalterung 5 einen ringförmigen äußeren Andruckrand 6 und einen zentralen Andruckbereich 7 auf, die über Stege 8 miteinander verbunden sind, wobei zwischen den Stegen 8 Durchbrechungen 9 ausgebildet sind, durch die ein Gasaustausch stattfinden kann.

Der Andruckrand 6 ist im Querschnitt U-förmig geformt und bildet somit eine Ringnut 10 aus, in die eine elastomere Ringdichtung 11 eingelegt ist.

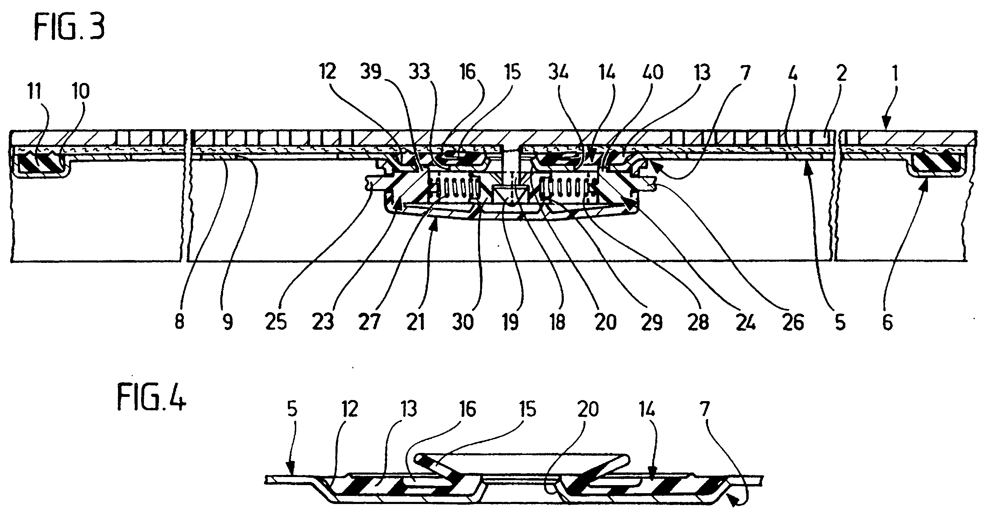

In den zentralen Andruckbereich 7 ist eine Ringnut 12 eingeformt, die einen Ringkörper 13 einer zentralen Dichtung 14 aufnimmt. An den Ringkörper 13 ist eine schräg zur Filterlage 4 hin abstehende, ringförmige Dichtlippe 15 angeformt, die elastisch gegen den Ringkörper 13 gebogen werden kann und dann in eine ringförmige Ausnehmung 16 des Ringkörpers 13 eintaucht (Figuren 3 und 4). Die Dichtlippe 15 kann somit einen relativ großen Dichtbereich überbrücken und sich an Filterlagen 4 unterschiedlicher Dicke anpassen.

Die Stege 8 verlaufen bei dem in den Figuren 1 bis 4 dargestellten Ausführungsbeispiel bogenförmig, wobei sie über ihre gesamte Länge eine Komponente in Umfangsrichtung, d.h. parallel zum Andruckrand 6, aufweisen, diese Komponente nimmt von innen nach außen zu.

Dieser Verlauf der Stege 8 mit einer deutlichen Komponente in Umfangsrichtung führt dazu, daß bei der Herstellung und auch beim späteren Betrieb mechanische Spannungen in der Filterhalterung 5 vermieden werden, sollten diese beispielsweise bei der Herstellung auftreten, können sie sich aufgrund des beschriebenen Verlaufes der Stege 8 ausgleichen.

Die Filterhalterung 5 kann beispielsweise als Tiefziehteil aus einer Blechplatte hergestellt werden, die Durchbrechungen 9 werden durch Stanzvorgänge erzeugt.

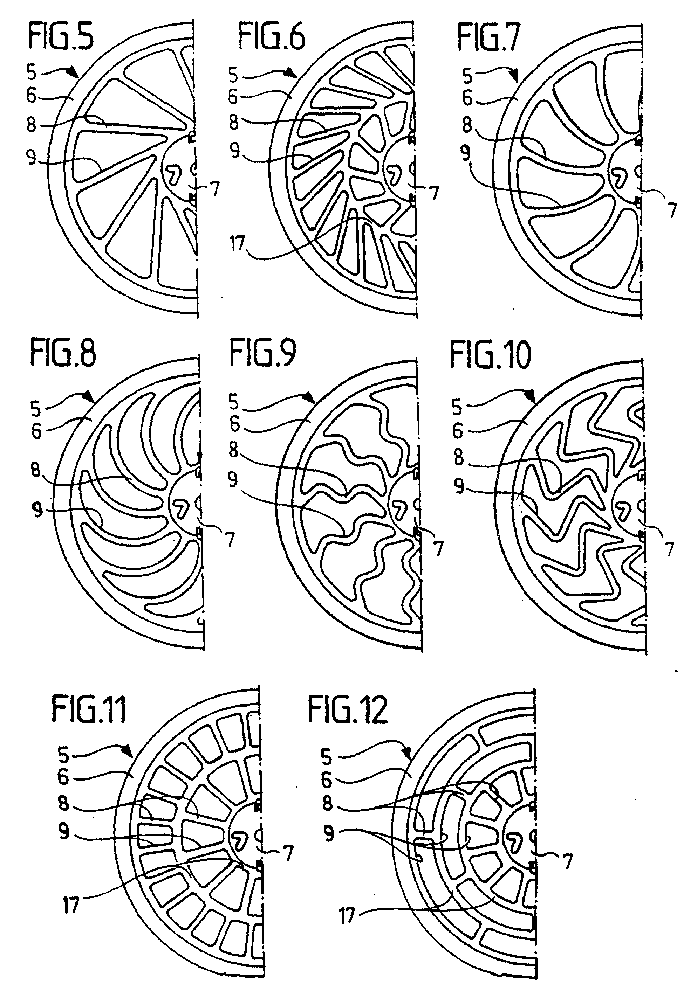

In den Figuren 5 bis 12 sind andere mögliche Verläufe der Stege 8 dargestellt. In Figur 5 verlaufen die Stege 8 von innen nach außen geradlinig, jedoch auch mit einer deutlichen Komponente in Umfangsrichtung. In dem Ausführungsbeispiel der Figur 6 sind ebenfalls geradlinig verlaufende Stege 8 vorgesehen, jedoch in diesem Falle zwei verschiedene Sätze, nämlich ein innerer Satz und ein äußerer Satz, die eine in Umfangsrichtung verlaufende Komponente mit entgegengesetzter Richtung aufweisen. Diese Stege 8 der beiden Sätze münden beide in einen konzentrisch zum äußeren Andruckrand 6 verlaufenden Mittelsteg 17 ein, und zwar derart, daß die Einmündungspunkte der Stege 8 des äußeren Satzes und des inneren Satzes in Umfangsrichtung gegeneinander versetzt sind. Damit ergibt sich eine Verbindung zwischen dem zentralen Andruckbereich 7 und dem äußeren Andruckrand 6, die im gesamten Bereich der Stege 8 eine deutliche Umfangskomponente aufweist.

Bei dem Ausführungsbeispiel der Figur 7 verlaufen die Stege 8 bogenförmig, wobei sie im randnahen Bereich eine Komponente in Umfangsrichtung aufweisen, deren Richtung verschieden ist von der Komponente im zentrumsnahen Bereich.

Beim Ausführungsbeispiel der Figur 8 verlaufen die Stege 8 in ähnlicher Weise wie beim Ausführungsbeispiel der Figuren 1 bis 4 bogenförmig, sie sind jedoch stärker gekrümmt als beim Ausführungsbeispiel der Figur 2, so daß die Stege 8 im wesentlichen tangential in den äußeren Andruckrand 6 einlaufen.

Beim Ausführungsbeispiel der Figur 9 sind die Stege 8 gewellt ausgeführt, d.h. es wechseln sich dadurch die Richtungen der Komponenten in Umfangsrichtung ab.

Dasselbe trifft zu beim Ausführungsbeispiel der Figur 10, bei dem die Stege 8 durch geradlinige, sich winklig aneinander anschließende Abschnitte gebildet werden.

Beim Ausführungsbeispiel der Figur 11 ist ähnlich wie beim Ausführungsbeispiel der Figur 6 ein konzentrischer Mittelsteg 17 vorgesehen, in den die Stege 8 versetzt zueinander einmünden. Im Gegensatz zum Ausführungsbeispiel der Figur 6 verlaufen die Stege 8 in diesem Falle genau radial, so daß die Komponente in Umfangsrichtung durch den Mittelsteg 17 gebildet wird. Die Verbindungslinien vom zentralen Andruckbereich 7 zum äußeren Andruckrand 6 verlaufen einmal über die radialen Stege 8 und zum anderen über den in Umfangsrichtung verlaufenden Mittelsteg 17, so daß diese Verbindungslinien insgesamt zumindest abschnittsweise Komponenten in Umfangsrichtung aufweisen, und diese Komponente in Umfangsrichtung stellt sicher, daß Verspannungen vermieden werden können.

Das Ausführungsbeispiel der Figur 12 entspricht weitgehend dem der Figur 11 mit dem Unterschied, daß in diesem Falle zwei konzentrische Mittelstege 17 vorgesehen sind, die über drei Sätze von radialen Stegen 8 miteinander verbunden sind. Auch in diesem Falle sind die Stege 8 in den Einmündungsbereichen in die Mittelstege 17 in Umfangsrichtung gegeneinander versetzt angeordnet.

Zur Festlegung der Filterlage 4 am Deckel 1 trägt dieser einen senkrecht abstehenden Zapfen 18 mit einem sich stufenförmig erweiternden Kopf 19. Dieser Zapfen 18 ragt durch eine zentrale Öffnung 20 im zentralen Andruckbereich 7 der Filterlage 4 hindurch und endet in einem topfförmigen Gehäuse 21, welches auf der der Filterlage 4 abgewandten Seite der Filterhalterung 5 mit dieser verbunden ist.

Das Gehäuse 21 nimmt in einer diametralen Führung 22 zwei längs dieser Führung verschiebbare Riegelkörper 23 und 24 auf, die in Form von Drucktasten 25 bzw. 26 aus dem Gehäuse 21 hervorstehen und die durch zwischen sich wirksame Druckfedern 27 und 28 in die maximal ausgeschobene Stellung verschoben werden. In dieser Stellung umgreifen beide Riegelkörper 23 und 24 mit einem Querarm 29, 30 den Zapfen 18 und untergreifen dadurch den stufig vorspringenden Kopf 19 des Zapfens 18. Dadurch wird das Gehäuse 21 mit der Filterhalterung 5 in axialer Richtung am Zapfen 18 und damit auch am Deckel 1 festgelegt, und zwar in einer Position, in der die Filterhalterung 5 gegen die Innenseite des Deckels 1 gespannt wird. Diese Stellung der Riegelkörper 23 und 24 wird daher als Verriegelungsstellung bezeichnet.

Beide Riegelkörper 23 und 24 können durch Druck auf die Drucktasten 25 und 26 gegen die Wirkungen der Druckfedern 27 und 28 in das Gehäuse 21 eingedrückt werden, dabei entfernen sie sich mit ihren Querarmen 29 und 30 von dem Zapfen 18 und ermöglichen somit, das Gehäuse 21 mit der Filterhalterung 5 vom Zapfen 18 abzunehmen und damit auch vom Deckel 1 zu entfernen. Diese Stellung der Riegelkörper 23 und 24 wird daher als Lösestellung bezeichnet.

Die Ausbildung ist dabei so gewählt, daß eine Abnahme des Gehäuses 21 und der Filterhalterung 5 vom Zapfen 18 nur möglich ist, wenn beide Riegelkörper 23 und 24 in gleicher Weise in das Gehäuse 21 eingeschoben sind, wenn nur ein Riegelkörper in die Lösestellung verschoben wird, ist eine Abnahme des Gehäuses und der Filterhalterung dagegen nicht möglich.

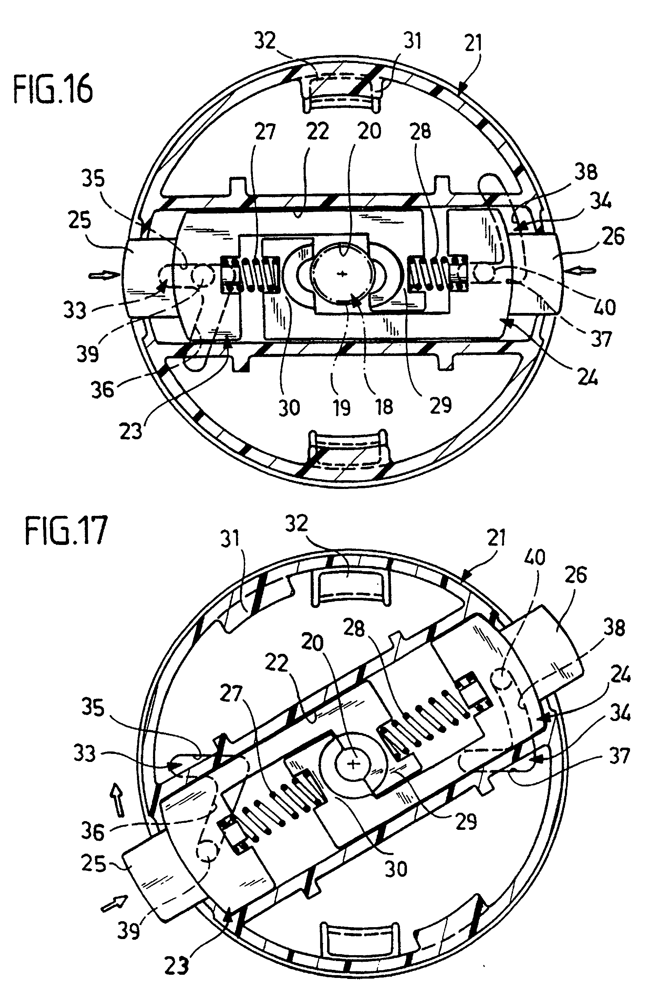

Das Gehäuse 21 mit den darin geführten Riegelkörpern 23 und 24 weist an seinem Innenmantel zwei nach innen ragende Vorsprünge 31 auf, die nach außen ragende Vorsprünge 32 an der Filterhalterung 5 untergreifen und dadurch das Gehäuse 21 an der Filterhalterung 5 festlegen. Der Eingriff der Vorsprünge 31 und 32 läßt sich dadurch lösen, daß das Gehäuse 1 um eine konzentrisch zum Zapfen 18 verlaufende Drehachse verdreht wird, sobald die Vorsprünge 31 und 32 dann nebeneinanderliegen, kann das Gehäuse 21 von der Filterhalterung 5 abgenommen werden (Figur 17). Es handelt sich hier also um eine Bajonettverriegelung zwischen Gehäuse 21 und Filterhalterung 5.

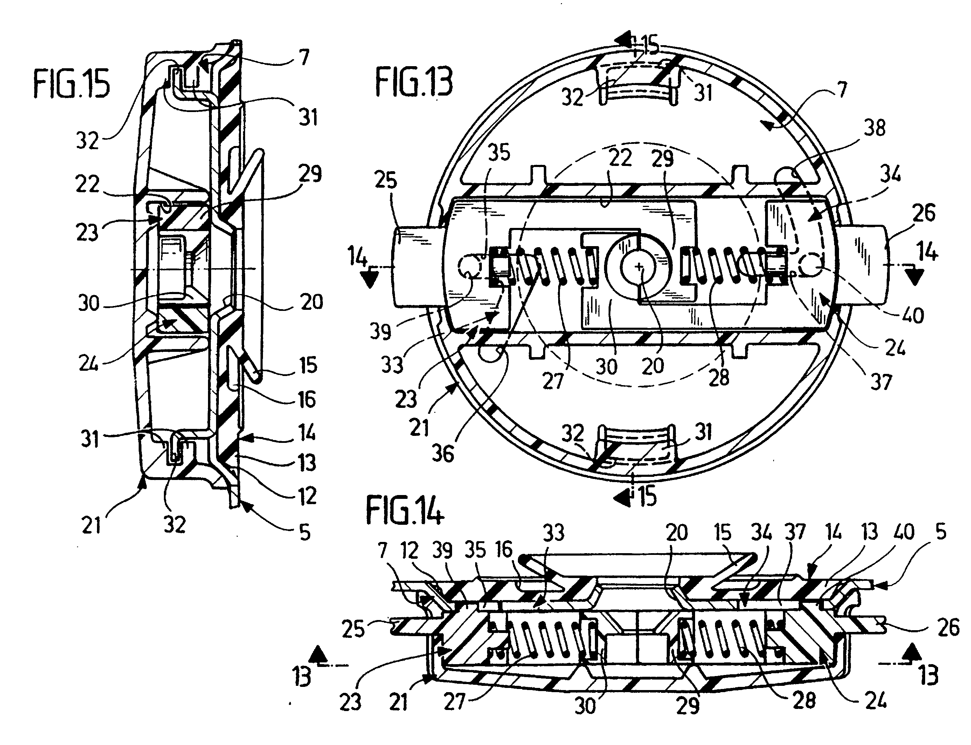

In dem zentralen Andruckbereich 7 der Filterhalterung 5 sind zwei Nockenbahnen 33 und 34 angeordnet, die einfach durch Schlitze im zentralen Andruckbereich 7 gebildet werden. Die Nockenbahn 33 weist einen von außen radial nach innen verlaufenden Abschnitt 35 und einen sich an dessen inneres Ende anschließenden, von dort schräg nach außen verlaufenden Abschnitt 36 auf, die andere Nockenbahn 34 einen ebenfalls radial nach innen verlaufenden Abschnitt 37 und einen sich an das äußere Ende dieses Abschnittes 37 anschließenden, in Umfangsrichtung verlaufenden Abschnitt 38. Dabei sind die radial nach innen verlaufenden Abschnitte 35 und 37 der beiden Nockenbahnen 33 bzw. 34 bezüglich dem Zapfen 18 spiegelsymmetrisch angeordnet, die Abschnitte 36 und 38 verlaufen in derselben Umfangsrichtung und enden beide in Positionen, die ebenfalls bezüglich des Zapfens 18 spiegelsymmetrisch angeordnet sind (Figur 13).

An ihrer Unterseite tragen beide Riegeikörper 23 und 24 zur offenen Seite des Gehäuses 21 hin gerichtete, nach unten abstehende Zapfen 39 bzw. 40, die in die Nockenbahnen 33 und 34 eintauchen.

Um das Gehäuse 21 mit der Filterhalterung 5 zu verbinden, wird zunächst das Gehäuse 21 mit den darin gelagerten Riegelkörpern 23 und 24 in einer Winkelstellung auf den zentralen Andruckbereich 7 der Filterhalterung 5 aufgesetzt, in der die beiden Zapfen 39 und 40 an den freien Enden der Abschnitte 36 bzw. 38 in diese eintauchen können (Figur 17). Bei dieser Winkelstellung liegen die Vorsprünge 31 und 32 nebeneinander und überdekken sich nicht.

Verdreht man das Gehäuse 21 um die zum Zapfen 18 konzentrische Drehachse, so werden die Vorsprünge 31 und 32 in ihre Überdeckungslage gebracht (Figur 16), d.h. der Bajonettverschluß wird verriegelt. Dies ist ohne weiteres möglich, bei dieser Drehbewegung läuft der Zapfen 40 des Riegelkörpers 24 in dem in Umfangsrichtung verlaufenden Abschnitt 38 der Nockenbahn 34, ohne daß der Riegelkörper 24 dabei relativ zum Gehäuse 21 verschoben wird. Der Zapfen 39 des Riegelkörpers 23 verläuft in dem schräg zur Mitte gerichteten Abschnitt 36 der Nockenbahn 33 und verschiebt dadurch den Riegelkörper 23 entgegen der Wirkung der beiden Druckfedern 27 und 28, bis das innen liegende Ende des radialen Abschnittes 35 der Nockenbahn 33 erreicht ist. Dann wird der Riegelkörper 23 durch die Druckfedern 27 und 28 radial nach außen verschoben, der Zapfen 39 bewegt sich dann im radialen Abschnitt 35 der Nockenbahn 33 nach außen und verriegelt dadurch das Gehäuse 21 gegen eine Verdrehung.

In dieser Position sind Gehäuse 21 und Filterhalterung 5 dauerhaft miteinander verbunden, wenn die beiden Riegelkörper 23 und 24 in der beschriebenen Weise gemeinsam in die Lösestellung verschoben werden, laufen die Zapfen 39 und 40 in den radialen Abschnitten 35 bzw. 37 der Nockenbahnen 33 und 34, und in jedem Falle wird dadurch das Gehäuse 21 gegen eine unerwünschte Drehung gesichert. In der Verriegelungsstellung erfolgt diese Drehsicherung durch den Riegelkörper 23, in der Lösestellung dagegen durch den Riegelkörper 24.

Um die Bajonettverbindung zwischen Gehäuse 21 und Filterhalterung 5 wieder lösen zu können, ist es notwendig, den Riegelkörper 23 allein in das Gehäuse 21 einzuschieben, um dadurch den Zapfen 39 des Riegelkörpers 23 bis an das innere Ende des Abschnittes 35 der Nokkenbahn 33 vorzuschieben. Erst dann ist ein Verdrehen des Gehäuses 21 möglich, das dann ohne weiteres soweit erfolgen kann, bis die Vorsprünge 31 und 32 wieder in der in Figur 17 dargestellten Weise nebeneinanderliegen und eine Abnahme des Gehäuses 21 möglich wird.

Durch die beschriebene Anordnung ist es unerheblich, welcher der beiden Riegelkörper 23, 24 mit welcher Nokkenbahn 33 bzw. 34 in Eingriff kommt. Das Gehäuse 21 und die Riegelkörper 23 und 24 sind einschließlich der Zapfen 39 und 40 vollständig symmetrisch aufgebaut, und die unterschiedliche Funktion der Riegelkörper 23 und 24 bei der Sicherung des Gehäuses 21 gegen eine Verdrehung erfolgt ausschließlich durch die unsymmetrische Anordnung der Nockenbahnen 33 und 34. Der Benutzer ist dadurch nicht gezwungen, beim Aufsetzen des Gehäuses 21 darauf zu achten, in welcher Richtung das Gehäuse 21 aufgesetzt wird, es genügt, wenn er beim Aufsetzen die Zapfen 39 und 40 in die Enden der Abschnitte 36 und 38 einsetzt.

## Patentansprüche

1. Tellerförmige Filterhalterung (5) für einen Sterilisierbehälter mit einem umlaufenden äußeren Andruckrand (6), einem zentralen Andruckbereich (7), einer Befestigungseinrichtung zur Festlegung der Filterhalterung (5) am Sterilisierbehälter und mit zwischen sich Durchbrechungen (9) frei lassenden Stegen (8) zwischen dem äußeren Andruckrand (6) und dem zentralen Andruckbereich (7), **dadurch gekennzeichnet, daß** die Stege (8; 17) zumindest abschnittsweise in einer Richtung verlaufen, die eine Komponente in Umfangsrichtung aufweist.

2. Filterhalterung nach Anspruch 1, **dadurch gekennzeichnet, daß** die Stege (8) zwischen dem zentralen Andruckbereich (7) und dem äußeren Andruckrand (6) bogenförmig verlaufen.

3. Filterhalterung nach Anspruch 1, **dadurch gekennzeichnet, daß** die Stege (8) zwischen dem zentralen Andruckbereich (7) und dem äußeren Andruckrand (6) geradlinig verlaufen.

4. Filterhalterung nach einem der voranstehenden Ansprüche, **dadurch gekennzeichnet, daß** die Stege (8) über ihre gesamte Länge mit einer in Umfangsrichtung gleichgerichteten Komponente verlaufen.

5. Filterhalterung nach einem der Ansprüche 1 bis 3, **dadurch gekennzeichnet, daß** die in Umfangsrichtung verlaufende Komponente des Steges (8) über dessen Länge ihre Richtung ändert.

6. Filterhalterung nach Anspruch 5, **dadurch gekennzeichnet, daß** die Stege (8) entlang ihrer Längsausdehnung wellen- oder zickzackförmig verlaufen.

7. Filterhalterung nach Anspruch 5, **dadurch gekennzeichnet, daß** die Stege (8; 17) entlang ihrer Längsausdehnung Abschnitte aufweisen, die abwechselnd radial und in Umfangsrichtung verlaufen.

8. Filterhalterung nach einem der voranstehenden Ansprüche, **dadurch gekennzeichnet, daß** benachbarte Stege (8) durch Verbindungsstege (17) miteinander verbunden sind.

9. Filterhalterung nach den Ansprüchen 7 und 8, **dadurch gekennzeichnet, daß** zwischen dem zentralen Andruckbereich (7) und dem äußeren Andruckrand (6) mindestens ein in Umfangsrichtung umlaufender Mittelsteg (17) angeordnet ist und daß zwischen diesem und dem äußeren Andruckrand (6), dem inneren Andruckbereich (7) oder einem benachbarten Mittelsteg (17) Verbindungsstege (8) mit radialer Komponente angeordnet sind, die auf gegenüberliegenden Seiten eines Mittelsteges (17) in Umfangsrichtung gegeneinander versetzt in diesen einmünden.

10. Filterhalterung nach Anspruch 9, **dadurch gekennzeichnet, daß** die Verbindungsstege (8) in radialer Richtung verlaufen.

11. Filterhalterung nach einem der voranstehenden Ansprüche, **dadurch gekennzeichnet, daß** am äußeren Andruckrand (6) eine Ringdichtung (11) gehalten ist.

12. Filterhalterung nach einem der voranstehenden Ansprüche, **dadurch gekennzeichnet, daß** am zentralen Andruckbereich (7) eine elastisch verformbare Andruckdichtung (14) angeordnet ist.

13. Filterhalterung nach Anspruch 12, **dadurch gekennzeichnet, daß** die Andruckdichtung (14) eine Lippendichtung mit einer elastisch an den zentralen Andruckbereich (7) andrückbaren Dichtlippe (15) ist.

14. Filterhalterung nach Anspruch 13, **dadurch gekennzeichnet, daß** die Lippendichtung (15) einen ringförmigen Lagerkörper (13) aufweist, an den die ringförmige Dichtlippe (15) angeformt ist.

15. Filterhalterung nach Anspruch 14, **dadurch gekennzeichnet, daß** der Lagerkörper (13) eine Ausnehmung (16) aufweist, in welche die Lippendichtung (15) eintaucht, wenn sie vollständig gegen den zentralen Andruckbereich (7) gedrückt wird.

16. Filterhalterung nach einem der voranstehenden Ansprüche, **dadurch gekennzeichnet, daß** die Befestigungseinrichtung ein Gehäuse mit zwei diametral federnd gegeneinander schiebbaren Riegelkörpern (23, 24) aufweist, die in einer unverschobenen Verriegelungsstellung ein die Filterhalterung (5) durchgreifendes Halteglied (18, 19) des Sterilisierbehälters (1) hintergreifen und dieses in einer verschobenen Lösestellung freigeben, daß das Gehäuse (12) an der Filterhalterung (5) im zentralen Andruckbereich (7) durch eine Drehbewegung festlegbar ist, bei der Vorsprünge (32) und Rücksprünge (31) an der Filterhalterung (5) bzw. am Gehäuse (21) sich hintergreifen, und daß mindestens eine lösbare Raste (23) vorgesehen ist, die bei an der Filterhalterung (5) festgelegtem Gehäuse (21) dieses gegen eine Verdrehung relativ zur Filterhalterung (5) sichert.

17. Filterhaiterung nach Anspruch 16, **dadurch gekennzeichnet, daß** die Raste durch einen der beiden Riegelkörper (23) gebildet wird, der das Gehäuse (21) in seiner Verriegelungsstellung gegen eine Verdrehung sichert und in seiner Lösestellung freigibt.

18. Filterhalterung nach Anspruch 17, **dadurch gekennzeichnet, daß** der andere Riegelkörper (24) das Gehäuse (21) in seiner Lösestellung gegen eine Verdrehung sichert und in seiner Verriegelungsstellung freigibt.

19. Filterhalterung nach einem der Ansprüche 17 oder 18, **dadurch gekennzeichnet, daß** Nockenbahnen (33, 34) und in diesen geführte Vorsprünge (39, 40) an dem zentralen Andruckbereich (7) bzw. den Riegelkörpern (23, 24) zusammenwirken und dadurch ein Verdrehen des Gehäuses (21) relativ zur Filterhalterung (5) nur in den bestimmten Stellungen der Riegelkörper (23, 24) erlauben.

20. Filterhalterung nach Anspruch 19, **dadurch gekennzeichnet, daß** eine Nockenbahn (33) für den das Gehäuse (21) in seiner Verriegelungsstellung sichernden Riegelkörper (23) einen radial nach innen gerichteten Abschnitt (35) und einen sich an dessen inneres Ende anschließenden, neben diesem Abschnitt (35) nach außen verlaufenden Abschnitt (36) aufweist.

21. Filterhalterung nach Anspruch 19 oder 20, **dadurch gekennzeichnet, daß** eine Nockenbahn (34) für den das Gehäuse (21) in seiner Freigabestellung sichernden Riegelkörper (24) einen radial nach innen gerichteten Abschnitt (37) und einen sich an dessen äußeres Ende anschließenden, in Umfangsrichtung verlaufenden Abschnitt (38) aufweist.

22. Filterhalterung nach den Ansprüchen 20 und 21, **dadurch gekennzeichnet, daß** die radial nach innen gerichteten Abschnitte (35, 37) der Nockenbahnen (33, 34) und die in die Nockenbahnen (33, 34) eingreifenden Vorsprünge (39, 40) bezüglich der Drehachse des Gehäuses (21) spiegelsymmetrisch angeordnet sind.

23. Filterhalterung nach Anspruch 22, **dadurch gekennzeichnet, daß** die Endpunkte der sich an die radial nach innen gerichteten Abschnitte (35, 37) anschließenden Abschnitte (36, 38) bezüglich der Drehachse des Gehäuses (21) spiegelsymmetrisch angeordnet sind.

## Claims

1. A plate-shaped filter holder (5) for a sterilisation container, comprising a circumferential outer pressure edge (6), a central pressure region (7), a fixing device for securing the filter holder (5) to the sterilisation container, and ribs (8) extending between the outer pressure edge (6) and the central pressure region (7) with openings (9) between the ribs (8), **characterised in that** the ribs (8; 17) extend at least in part in a direction having a circumferential component.

2. A filter holder according to claim 1, **characterised in that** the ribs (8) extend in a curved manner between the central pressure region (7) and the outer pressure edge (6).

3. A filter holder according to claim 1, **characterised in that** the ribs (8) extend in a linear manner between the central pressure region (7) and the outer pressure edge (6).

4. A filter holder according to any one of the preceding claims, **characterised in that** the ribs (8) extend over their entire length with an equidirectional circumferential component.

5. A filter holder according to any one of claims 1 to 3, **characterised in that** the circumferential component of the rib (8) changes direction over its length.

6. A filter holder according to claim 5, **characterised in that** the ribs (8) extend in an undulating or zigzag manner along their longitudinal extent.

7. A filter holder according to claim 5, **characterised in that** the ribs (8; 17) have portions along their longitudinal extent which extend alternately radially and circumferentially.

8. A filter holder according to any one of the preceding claims, **characterised in that** adjacent ribs (8) are connected to one another by connecting ribs (17).

9. A filter holder according to claims 7 and 8, **characterised in that** at least one circumferentially extending intermediate rib (17) is arranged between the central pressure region (7) and the outer pressure edge (6) and **in that** connecting ribs (8) with a radial component are arranged between the intermediate rib (17) and the outer pressure edge (6), the inner pressure region (7) or an adjacent intermediate rib (17) and, on opposite sides of an intermediate rib (17), join the latter in a staggered arrangement in the circumferential direction.

10. A filter holder according to claim 9, **characterised in that** the connecting ribs (8) extend radially.

11. A filter holder according to any one of the preceding claims, **characterised in that** a ring seal (11) is held on the outer pressure edge (6).

12. A filter holder according to any one of the preceding claims, **characterised in that** an elastically deformable pressure seal (14) is arranged on the central pressure region (7).

13. A filter holder according to claim 12, **characterised in that** the pressure seal (14) is a lip seal with a sealing lip (15) resiliently pressable against the central pressure region (7).

14. A filter holder according to claim 13, **characterised in that** the lip seal (15) has an annular bearing body (13) on which the annular sealing lip (15) is formed.

15. A filter holder according to claim 14, **characterised in that** the bearing body (13) has a recess (16) into which the lip seal (15) extends when it is pressed fully against the central pressure region (7).

16. A filter holder according to any one of the preceding claims, **characterised in that** the fixing device comprises a housing with two locking members (23, 24) which are resiliently and diametrically pushable towards one another, engage behind a holding member (18, 19) of the sterilisation container (1), extending through the filter holder (5), in an undisplaced locking position and release the holding member (18, 19) in a displaced release position, **in that** the housing (12) is securable to the filter holder (5) in the central pressure region (7) by a rotation movement, during which projections (32) and recesses (31) on the filter holder (5) and the housing (21) respectively engage behind one another, and **in that** at least one releasable detent (23) is provided which secures the housing (21) against rotation relative to the filter holder (5) when the housing (21) is secured thereto.

17. A filter holder according to claim 16, **characterised in that** the detent is formed by one of the two locking members (23), which secures the housing (21) against rotation in its locking position and releases it in its release position.

18. A filter holder according to claim 17, **characterised in that** the other locking member (24) secures the housing (21) against rotation in its release position and releases it in its locking position.

19. A filter holder according to either one of claims 17 and 18, **characterised in that** cam paths (33, 34) and projections (39, 40) guided therein co-operate on the central pressure region (7), or rather the locking members (23, 24), and thereby allow rotation of the housing (21) relative to the filter holder (5) only in the specified positions of the locking members (23, 24).

20. A filter holder according to claim 19, **characterised in that** a cam path (33) for the locking member (23) securing the housing (21) in its locking position has a portion (35) extending radially inwards and a portion (36) adjoining the inner end of the latter and extending outwards adjacent to this portion (35).

21. A filter holder according to claim 19 or 20, **characterised in that** a cam path (34) for the locking member (24) securing the housing (21) in its release position has a portion (37) extending radially inwards and a portion (38) adjoining the outer end of the latter and extending circumferentially.

22. A filter holder according to claims 20 and 21, **characterised in that** the portions (35, 37) of the cam paths (33, 34) extending radially inwards and the projections (39, 40) engaging in the cam paths (33, 34) are arranged mirror-symmetrically relative to the rotation axis of the housing (21).

23. A filter holder according to claim 22, **characterised in that** the end points of the portions (36, 38) adjoining the portions (35, 37) extending radially inwards are arranged mirror-symmetrically relative to the rotation axis of the housing (21).

## Revendications

1. Support de filtre (5) en forme d'assiette pour un récipient de stérilisation, comportant un bord de pression extérieur (6) périphérique, une région de pression centrale (7), un dispositif de fixation pour immobiliser le support de filtre (5) sur le récipient de stérilisation et comportant, entre le bord de pression extérieur (6) et la région de pression centrale (7), des entretoises (8) laissant libres des percées (9), **caractérisé en ce que** les entretoises (8 ; 17) s'étendent au moins par tronçons dans une direction qui présente une composante en direction périphérique.

2. Support de filtre selon la revendication 1, **caractérisé en ce que** les entretoises (8) s'étendent en forme arquée entre la région de pression centrale (7) et le bord de pression extérieur (6).

3. Support de filtre selon la revendication 1, **caractérisé en ce que** les entretoises (8) s'étendent rectilignes entre la région de pression centrale (7) et le bord de pression extérieur (6).

4. Support de filtre selon l'une des revendications précédentes, **caractérisé en ce que** sur toute leur longueur, les entretoises (8) s'étendent avec une composante dirigée dans la même direction que la direction périphérique.

5. Support de filtre selon l'une des revendications 1 à 3, **caractérisé en ce que** la composante s'étendant en direction périphérique, de l'entretoise (8), change sa direction sur la longueur de l'entretoise.

6. Support de filtre selon la revendication 5, **caractérisé en ce que** les entretoises s'étendent en forme d'ondulations ou de zigzags le long de leur extension longitudinale.

7. Support de filtre selon la revendication 5, **caractérisé en ce que** les entretoises (8 ; 17) présentent le long de leur extension longitudinale des tronçons qui s'étendent en alternance en direction radiale et en direction périphérique.

8. Support de filtre selon l'une des revendications précédentes, **caractérisé en ce que** des entretoises voisines (8) sont reliées les unes aux autres par des entretoises de liaison (17).

9. Support de filtre selon les revendications 7 et 8, **caractérisé en ce qu'**entre la région de pression centrale (7) et le bord de pression extérieur (6) est agencée au moins une entretoise médiane (17) s'étendant en direction périphérique et **en ce qu'**entre celle-ci et le bord de pression extérieur (6), la région de pression intérieure (7) ou une entretoise médiane voisine (17), sont agencées des entretoises de liaison (8) avec composante radiale qui sont décalées les unes par rapport aux autres en direction périphérique sur des côtés opposés d'une entretoise médiane (17) et qui débouchent dans celle-ci.

10. Support de filtre selon la revendication 9, **caractérisé en ce que** les entretoises de liaison (8) s'étendent en direction radiale.

11. Support de filtre selon l'une des revendications précédentes, **caractérisé en ce qu'**un joint annulaire (11) est maintenu sur le bord de pression extérieur (6).

12. Support de filtre selon l'une des revendications précédentes, **caractérisé en ce que** sur la région de pression centrale (7) est agencé un joint de pression (14) élastiquement déformable.

13. Support de filtre selon la revendication 12, **caractérisé en ce que** le joint de pression (14) est un joint à lèvre avec une lèvre d'étanchéité (15) qui peut être pressée de manière élastique sur la région de pression centrale (7).

14. Support de filtre selon la revendication 13, **caractérisé en ce que** le joint à lèvre (15) présente un corps de palier annulaire (13) sur lequel est formée la lèvre d'étanchéité (15) annulaire.

15. Support de filtre selon la revendication 14, **caractérisé en ce que** le corps palier (13) présente un évidement dans lequel plonge le joint à lèvre (15) lorsqu'il est complètement pressé contre la région de pression centrale (7).

16. Support de filtre selon l'une des revendications précédentes, **caractérisé en ce que** le dispositif de fixation présente un boîtier avec deux corps de verrouillage (23, 24) déplaçables diamétralement l'un par rapport à l'autre de manière élastique qui, dans une position de verrouillage non déplacée, engagent par l'arrière un organe de retenue (18, 19) du réservoir de stérilisation (1), qui traverse le support de filtre (5) et libère ledit organe de retenue dans une position de desserrage déplacée, **en ce que** le boîtier (21) peut être immobilisé sur le support de filtre (5) dans la région de pression centrale (7) par un mouvement de rotation au cours duquel des saillies (32) et des retraits (31) sur le support de filtre (5) et sur le boîtier (21), respectivement, s'engagent par l'arrière, et **en ce qu'**il est prévu au moins un cran (23) détachable qui assure le boîtier (21) à l'encontre d'une rotation par rapport au support de filtre (5) lorsque le boîtier est immobilisé sur le support de filtre (5).

17. Support de filtre selon la revendication 16, **caractérisé en ce que** le cran est formé par un des deux corps de verrouillage (23) qui assure le boîtier (21) dans sa position de verrouillage à l'encontre d'une rotation et qui le libère dans sa position de desserrage.

18. Support de filtre selon la revendication 17, **caractérisé en ce que** l'autre corps de verrouillage (24) assure le boîtier (21) dans sa position de desserrage à l'encontre d'une rotation et le libère dans sa position de verrouillage.

19. Support de filtre selon l'une ou l'autre des revendications 17 et 18, **caractérisé en ce que** des pistes de came (33, 34) et des saillies (39, 40) guidées dans celles-ci coopèrent au niveau de la région de pression centrale (7) et des corps de verrouillage (23, 24), respectivement, et ne permettent ainsi une rotation du boîtier (21) par rapport au support de filtre (5) que dans les positions déterminées des corps de verrouillage (23, 24).

20. Support de filtre selon la revendication 19, **caractérisé en ce que** pour le corps de verrouillage (23) assurant le boîtier (21) dans sa position de verrouillage, une piste de came (33) présente un tronçon (35) dirigé radialement vers l'intérieur et un tronçon (36) s'étendant vers l'extérieur à côté de ce tronçon (35) et se raccordant à son extrémité intérieure.

21. Support de filtre selon l'une ou l'autre des revendications 19 et 20, **caractérisé en ce qu'**une piste de came (34) pour le corps de verrouillage (24) assurant le boîtier (21) dans sa position de libération présente un tronçon (37) dirigé radialement vers l'intérieur et un tronçon (38) s'étendant en direction périphérique et se raccordant à son extrémité extérieure.

22. Support de filtre selon les revendications 20 et 21, **caractérisé en ce que** les tronçons (35, 37) dirigés radialement vers l'intérieur des pistes de cames (33, 34) et les saillies (39, 40) s'engageant dans les pistes de cames (33, 34) sont agencés symétriquement par rapport à l'axe de rotation du boîtier (21).

23. Support de filtre selon la revendication 22, **caractérisé en ce que** les points terminaux des tronçons (36, 38) qui se raccordent aux tronçons (35, 37) dirigés radialement vers l'intérieur sont agencés symétriquement par rapport à l'axe de rotation du boîtier (21).
